# EUROPEAN PATENT SPECIFICATION

(11) **EP 2 415 404 B1**
(45) Date of publication and mention of the grant of the patent: **30.08.2017**
(21) Application number: 10305866.5
(22) Date of filing: 06.08.2010
(51) Int. Cl.: A61B 17/04

(54) **Arthroscopic device for cutting a suture and arthroscopic surgical kit including such a device**
Arthroskopievorrichtung zum Schneiden einer Naht und chirurgisches Arthroskopiekit, das eine solche Vorrichtung beinhaltet
Dispositif arthroscopique pour couper une suture et kit chirurgical arthroscopique incluant ledit dispositif

(43) Date of publication of application: 08.02.2012
(73) Proprietor: Tornier, Inc., Bloomington, MN 55437 (US)
(72) Inventor: MORGAN, Daniel, SALAM - (MA), MA 01970 (US); SNELL, Douglas, AMESBURY - (MA), MA 01913 (US)
(74) Representative: Lavoix

(56) References cited:
- EP-A1- 2 098 171
- WO-A1-2010/042845
- US-A- 3 995 619
- US-A1- 2007 106 310

## Description

The present invention relates to an arthroscopic device for cutting a suture. The invention relates also to a surgical kit including such a device.

The invention may be used in particular, but not exclusively, during an arthroscopic surgical procedure In the joint space of a human shoulder.

In such a surgical procedure, anchors are often implanted to secure a graft or a synthetic endoprosthesis, such as a patch, to a bone. For this purpose, sutures are fixedly connected to the anchors, after having been threaded through at least the graft or the endoprosthesis. At the end of the procedure, the free tails of theses sutures need to be cut because they constitute foreign bodies in the joint space.

US-A-2005/0234481 discloses arthroscopic devices which may be used to cut such suture tails. According to one embodiment disclosed in this document, which corresponds to the preamble of appended claim 1, the device comprises a rod including a J-hook shaped distal end part provided with a cutting blade located in the middle of the bottom of the J-shape. A suture to be cut is placed transversally to the rod, along the bottom of the J-shape. In view of tautening or tightening the suture across the blade, a tubular body is slidably moved in frictional contact around the rod, up to pinch the suture against the bottom of the J-shape, at two radially opposed points of the periphery of the rod. In practice, the surgeon has to be extremely careful for firmly tautening or tightening the suture before pinching it by the tubular body, otherwise there is a risk for the suture to be only partially cut by the blade. In other words, to be efficient, this device requires a high level of skill and dexterity, especially under the arthroscopic conditions of use. Besides, due to the structure of this device, a significant length of the suture necessarily remains in place after cutting. In particular, such quite long tails may protrude from a bone hole where an anchor linked to the suture is implanted. However, these long suture tails present an abrasion material in the tightly spaced bone joint, which is prejudicial for the quality of the arthroscopic procedure.

U5-A-20071106310, on which the preamble of claim 1 is based, discloses an arthroscopic device for cutting a stainless steel suture. Rigidity of such a metallic suture is utilized to, substantially in the same time, both cut a part of the suture by a cutting rod moveable within a tubular member through which the suture is initially loaded, and retain another part of the suture between the cutting rod and the member. In practice, as the cutting area is within a non-ended part of the fixed outer member, this device leaves necessarily a significant length of suture in place after cutting.

An object of the present invention is to propose an arthroscopic cutter device, which eliminates the free suture tails as far as possible, in a simple and efficient manner.

To this end, the subject of the invention is an arthroscopic device for cutting a suture, as defined in appended claim 1. Another subject of the invention is an arthroscopic surgical kit as defined in appended claim 14. Additional advantageous features of this device or of this kit are specified in dependent claims 2 to 13 and 15.

One of the ideas underlying the invention is to provide a radial tolerance between the inner member and the outer body of the device and to thread the suture to be cut in a radial gap between one side of the inner member and the outer body, due to the aforesaid tolerance, in order to induce a tension action on this suture, and, by that way, to use this suture to bias the two components when they are moved to each other for cutting the suture. Thus, the cutter action is advantageously performed with a "clearance zero" between the opposed side of the inner member and a facing portion of the outer body.

For the surgeon, the device according to the invention is easy to use and efficient. After having placed a suture to be cut in the distal hole of the inner member, the distal end part of this member is placed where the cutting is desired, especially as near as possible to the structure from which the suture protrudes, in particular as near as possible to a proximal end of a bone anchor from which the suture extends. Then, after having pulled on the suture to reduce its length between the device and the aforesaid structure, the surgeon controls the device to move the outer body from its load position to its cut position: thanks to the device, the suture is used to provide a zero clearance radial fit between the inner member and the outer body when these two components interfere for cutting the suture. After the cutting, the remaining part of the suture is as short as possible. In particular, when the distal end part of the device is introduced in a bone hole for cutting a suture extending from this bone hole, the remaining part of the cut suture is advantageously located within the bone hole, below the bone surface. In other words, in this case, the device according to the invention eliminates the suture tail from a bone joint space. Of course, in this use context, any distal geometry of the device, which fits inside a bone hole or a bone tunnel, is conceivable.

Embodiments of the invention will be better understood from reading the description which will follow, which is given solely by way of example and with reference to the drawings in which:
- figure 1 is a schematic elevation view of a device according to an embodiment of the invention, which is shown in a rest configuration;
- figure 2 is a perspective view of a distal part of the device of figure 1;
- figure 3 is a view in section in plane III of figure 2;
- figure 4 is a view in section along line IV-IV of figure 3;
- figures 5, 6 and 7 are views respectively similar to figures 1, 2 and 4, showing the device in a load configuration and including sutures to be cut by the device;
- figures 8, 9 and 10 are views respectively similar to figures 5, 6 and 7, showing the device in a slide configuration and including a bone anchor to which the sutures are connected;
- figure 11 is a view similar to figure 10, showing a relative movement between the sutures and the components of the device in its slide configuration; and
- figures 12, 13 and 14 are views respectively similar to figures 8, 9 and 10, showing the device in a cut configuration.

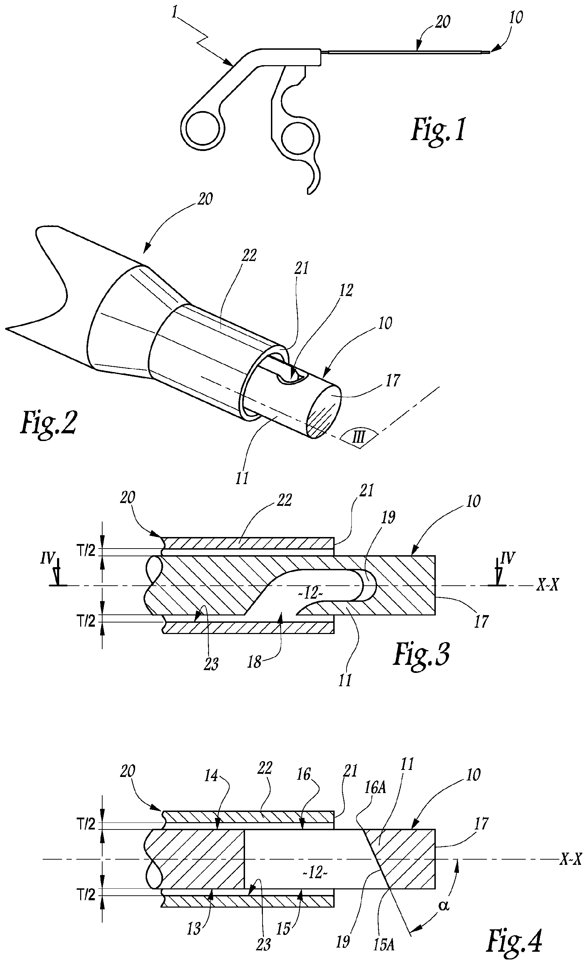

Figures 1 to 4 depict an arthroscopic cutter device 1 comprising two main components which are an inner rod 10 and an outer tube 20. Rod 10 extends in length about a central axis X-X. In the shown embodiment, rod 10 is cylindrical, with a circular base centered on axis X-X.

Tube 20 is arranged around rod 10, extending along substantially all the length of the rod, except at a distal end part 11 of rod 10, which emerges from a distal end 21 of tube 20, as clearly shown in figures 2 to 4. Tube 20 is substantially coaxial with rod 10 and has a cross section which internally corresponds to the cross section of rod 10, with interposition between them of a non-zero radial tolerance T: in figures 3 and 4, this tolerance T is uniformly distributed around rod 10, which explains why an half of tolerance T is found between the rod and each of the two radially opposed portions of tube represented in section.

Thus, in the shown embodiment, tube 20 has a cross section which is internally circular and externally circular too.

In use, tube 20 is intended to be axially moved around rod 10, by sliding on this rod. By that way, distal end part 21 of tube 20 is able to cover a variable length of distal end part 11 of rod 10, as better explained hereinafter.

In order to control the relative movement between rod 10 and tube 20, device 1 comprises appropriate control means. In the shown embodiment, a handle 31 is fixedly connected to a proximal part of rod 10 and a lever 32 is mounted in an articulated manner to this handle, so that, when rocked around an axis perpendicular to axis X-X, the lever transmits force and motion to tube 20 with respect to rod 10. In practice, different transmission mechanisms may be arranged between tube 20 and an actuator such as lever 32.

In any case, the aforesaid control means are adapted to be actuated outside a human body while a distal part of rod 10, including its end part 11, and a distal part of tube 20, including an end part 22 thereof, are placed in a bone joint space, after having been introduced via an arthroscopic cannula. In practice, the outside diameter of tube 20 is accordingly dimensioned.

As best shown in figures 2 to 4, end part 11 of rod 10 is provided with a through hole 12 which extends transversally to axis X-X. More precisely, this hole 12 extends between two radially opposed sides 13 and 14 of rod 10, joining two free openings 15 and 16 delimited by rod 10, respectively on side 13 and on side 14 thereof. As clearly shown in figure 4, the openings 15 and 16 are not strictly aligned, in the sense that, along axis X-X, opening 15 presents a distal end point 15A which is nearer to the distal end 17 of rod 10 than the distal end point 16A of opening 16. In other words, the line extending through the aforesaid two distal end points 15A and 16A is not perpendicular to axis X-X, but defines with respect to this axis X-X an angle α different from 90°. By that way, insofar as distal end 21 of tube 20 lies in a geometrical plane perpendicular to axis X-X, it will be understood that, when tube is axially moved around rod 10 in direction to distal end 17 of the rod, end part 22 of the tube progressively overlaps the openings 15 and 16, but covers in totality opening 16 before covering in totality opening 15, because, along axis X-X, its end 21 radially faces end point 16A before radially facing end point 15A. The interest of this feature will be explained hereinafter.

Furthermore, in the embodiment shown in the figures, end part 11 of rod 10 is shaped in the form of a J-hook which implies that, in addition to its two opposed openings 15 and 16, hole 12 is also open on one of the two sides which link to each other the sides 13 and 14 around rod 10. In other words, as shown in figure 3, hole 12 is peripherally delimited by a wall the distal portion 19 of which joints the openings 15 and 16 whereas the proximal portion thereof is broken by an opening 18 which is defined in one of the aforesaid two sides of rod 10, distinctively from the openings 15 and 16. In order to obtain the J-shape, this opening 18 is axially offset with respect to the openings 15 and 16, being situated farer from distal end 17 of rod 10 than these openings 15 and 16. The interest of this J-shape will be explained hereinafter.

An example of the use of device 1 will be now explained.

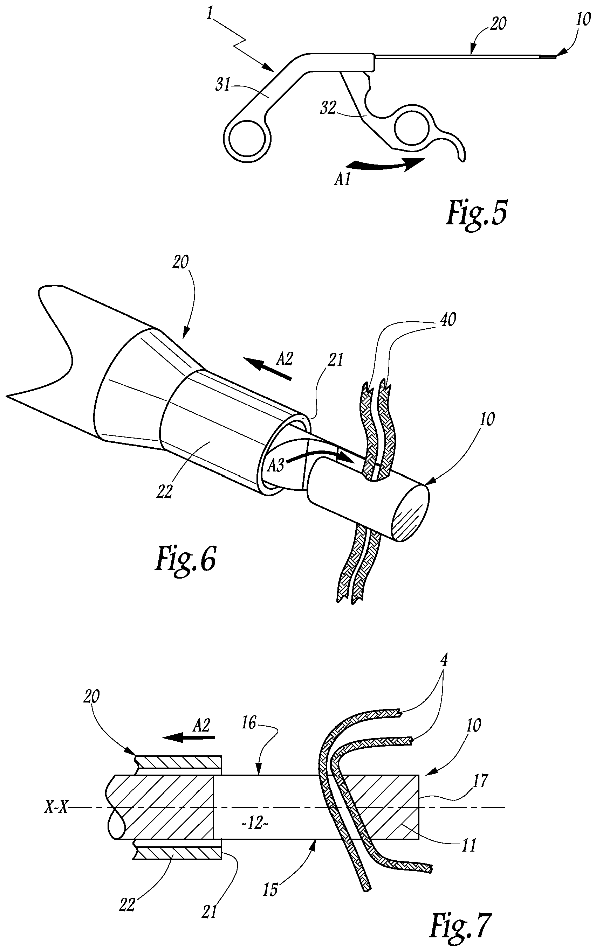

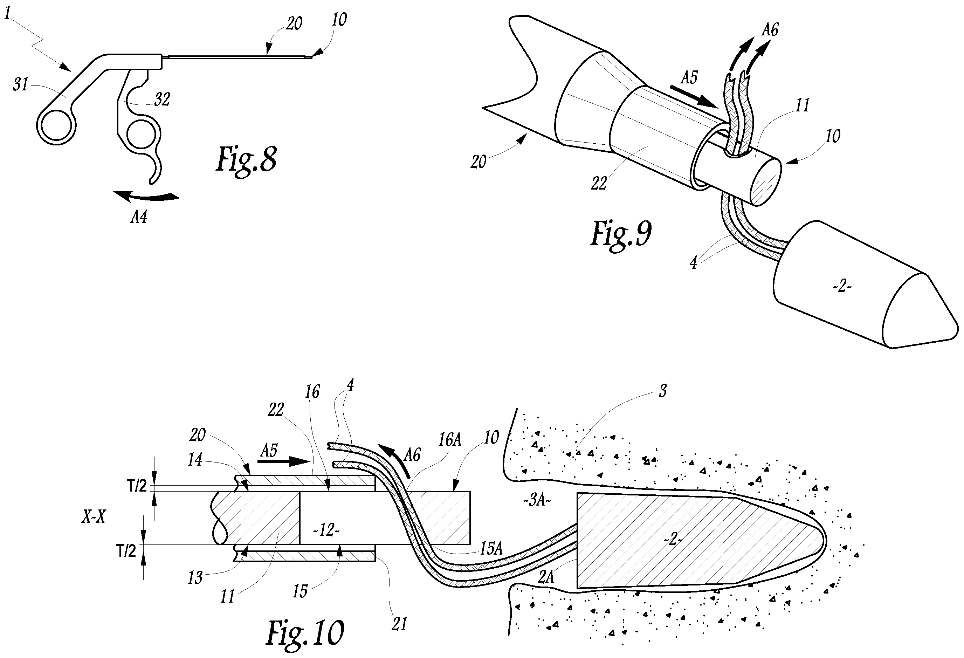

In a first step, whereas device 1 is at rest as shown in figures 1 to 4, the distal parts of rod 10 and tube 20 are introduced in a bone joint space, for example in a glenohumeral space of a human shoulder. Beforehand, an arthroscopic surgical procedure may have been performed, for example during which a bone anchor was implanted in one of the bones defining the joint space, this bone anchor and the aforesaid bone being respectively referenced 2 and 3 on figures 10, 11 and 14. As shown in the aforesaid figures, the proximal end 2A of anchor 2 is below the surface of bone 3, which keeps free a sub-cortical volume 3A in bone 3, corresponding to the proximal part of the hole where anchor 2 is implanted. Besides, as shown in the aforesaid figures, anchor 2 is non-removably connected to two sutures 4 shown in figures 6 and 7. In practice, the connection between the anchor and these sutures 4 may be realized by different ways: for example, each of these sutures is locked by a locking mechanism integrated in the anchor, or is pinched between the external face of the anchor and the wall of the bone hole in which the anchor is implanted.

In a second step shown in figures 5 to 7, the surgeon rocks lever 32 opposite of handle 31, as indicated by an arrow A1 in figure 5. By that way, tube 20 is driven in a proximal direction around rod 10, as indicated by an arrow A2 in figures 6 and 7. Thus, tube 10 is moved from its rest position of figures 1 to 4, in which end part 22 of the tube covers the opening 18, to a load position shown in figures 5 to 7, in which end part 22 reveals this opening 18. By that way, hole 12 is free to receive the sutures 4 which are advantageously introduced within hole 12 through opening 18 then axially driven to the distal portion 19 of this hole, i.e. to the bottom of the J-shape of end part 11 of rod 10, as indicated by an arrow A3 in figure 6.

Of course, in the aforesaid load position, distal end 21 of tube 10 is sufficiently axially away from hole 12 to keep free the two main opposed openings 15 and 16 of hole 12. That is the reason why, according to a not shown embodiment, the J-hook shape of end part 11 of rod 10 may be replaced by an eyelet shape, that is to say that, in end part 11 of rod 10, a continuous wall may peripherically delimit a hole similar to hole 12, which is open only at two opposed openings similar to the openings 15 and 16. In that case, sutures 4 are threaded in this eyelet hole.

In a third step shown in figures 8 to 11, the surgeon rocks lever 32 towards handle 31, as indicated by an arrow A4 in figure 8. By that way, tube 20 is driven axially around rod 10 in a distal direction, as indicated by an arrow A5 in figures 9 to 11. Thus, tube 20 moves from its load position shown in figures 5 to 7 to a first intermediate position shown in figures 8 to 10, in which end part 22 of the tube progressively covers the openings 15 and 16. In the same step, the surgeon moves device 1 nearer to bone anchor 2, especially by substantially aligning distal end 17 of rod 10 with subcortical volume 3A. Of course, surgeon progressively pulls on sutures 4, as indicated by an arrow A6 in figures 9 and 10, in order to progressively increase the length of the part of the sutures, protruding from opening 16, which reduces quite as much the length of the part of the sutures, extending from anchor 2 to opening 15.

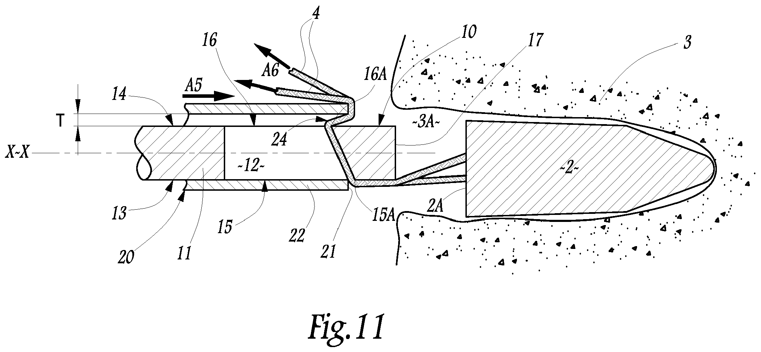

In the aforesaid first intermediate position of tube 20, the sutures 4 do not interfere with the tube, in the sense that, even if end part 22 of tube 20 covers in part the openings 15 and 16, distal end 21 of the tube is sufficiently proximal away from the distal end points 15A and 16A of these openings to allow the sutures 4 to extend within hole 12, from side 13 of rod 10 to side 14 of this rod without contacting the inner face 23 of tube 20. That is the reason why, as shown in figure 10, radial tolerance T between rod 10 and tube 20 is distributed in a uniform manner around the rod, typically by half on side 13 and on side 14. However, when tube 20 continues its distal sliding around rod 10 due to the rocking of lever towards handle 31, the tube reaches a subsequent intermediate position shown in figure 11. In this intermediate position of figure 11, distal end 21 of tube 20 is distal offset with respect to distal end point 16A of opening 16 along axis X-X, that is to say that, along this axis, distal end 21 is nearer to distal end 17 of rod 10 than distal end point 16A of opening 16. In other words, end part 22 of tube 20 axially covers the whole opening 16. At the same time, opening 15 is still partly open, because distal end 21 of tube 20 is proximal offset with respect to distal end point 15A of this opening 15. In this intermediate position of figure 11, the arrangement of the sutures 4 through opening 15 is unchanged with respect to figure 10. However, on side 14 of rod 10 in figure 11, the path of the sutures 4 is modified because the sutures 4 need to go around distal end 21 of tube 20. For this purpose, the part of each suture 4, emerging from opening 16, slips radially between end part 11 of rod 10 and end part 22 of tube 20, that is to say slips into a radial gap 24 defined between inner face 23 of end part 22 of the tube and side 14 of end part 11 of the rod. Of course, this radial gap 24 is due to the radial tolerance T provided between rod 10 and tube 20.

In practice, one of ordinary skill in the art will understand that, when tube 20 is moved from its position of figure 10 to its position of figure 11, the sutures 4 induce a biasing action between rod 10 and tube 20. Indeed, when distal end 21 of tube 20 is very near to distal end point 16A of opening 16, the suture part axially interposed between them tends to control a transversal movement of tube 20 with respect to rod 10, according to which, on side 14 of rod 10, inner face 23 of the tube radially moves away from the rod whereas, on side 13 of rod 10, inner face 23 of the tube radially moves closer to the rod. Besides, due to the strength of the sutures 4 and/or the fact that these sutures are sufficiently tightened by the surgeon, the part of the sutures, passing through gap 24, radially enlarges this gap as much as possible, that is to say until the radial dimension of this gap corresponds to the total value of tolerance T, as shown in figure 11. By that way, on side 13 of rod 10, inner face 23 of the tube contacts the rod without radial clearance.

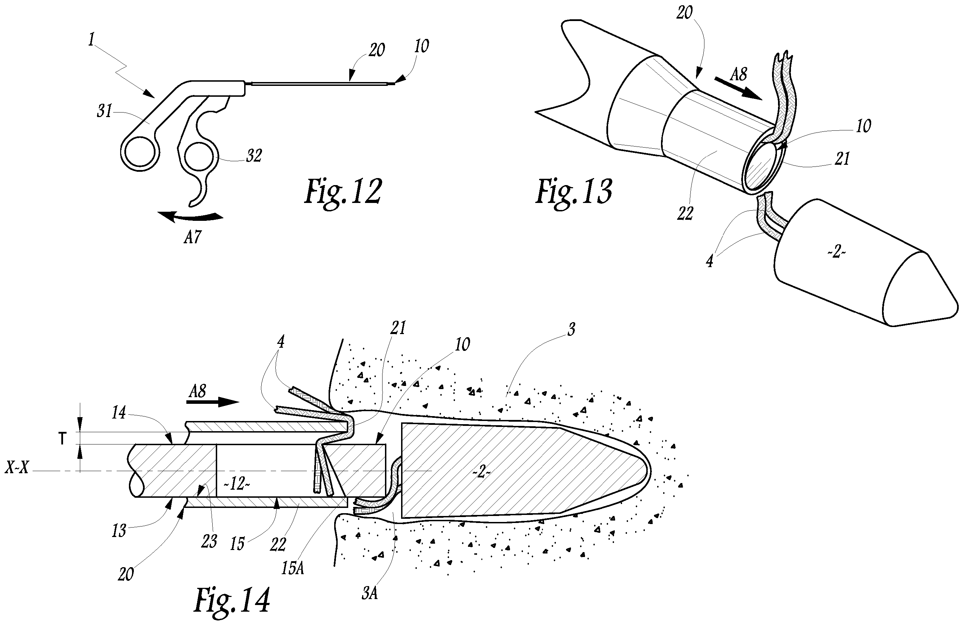

In a fourth step, as the surgeon continues to rock lever 32 towards handle 31 as indicated by an arrow A7 in figure 12, tube 20 continues to be axially moved in a distal direction, as indicated by an arrow A8 in figures 13 and 14, and reaches a cut position shown in figures 12 to 14. In this cut position, end part 22 of tube 20 axially covers the whole opening 15, that is to say that distal end 21 of tube 20 axially reaches distal end point 15A of opening 15. As inner face 23 of tube 20 contacts side 13 of hole 10 without clearance, the portion of distal end 21 of the tube, radially facing distal end point 15A of opening 15, interferes with this end point, with interposition of the sutures 4 which are cut by this mechanical interference between rod 10 and tube 20. In practice, the cutting action may be performed by a cutting edge located at distal end point 15A of opening 15, more generally located in a distal portion of this opening 15. Another solution consists to arrange a cutting edge in the portion of distal end 21 of tube 20, which radially faces side 13 of rod 10. In that case, tube 10 is preferably fixed in rotation around axis X-X with respect to rod 10. Of course, two cutting edges may be provided respectively at opening 15 and at distal end 21 of tube.

Advantageously, the biasing action provided by the sutures 4 is adjusted thanks to an appropriate gauging of device 1. In particular, the aforesaid tolerance T and angle α are chosen so that the non-cutting side 14 of rod 10 engages tube 20 before the cutting side 13 does to ensure the tolerance T is removed from the rod and the tube at this side 13.

Furthermore, the surgeon drives tube 20 up to its cut position, after having put in place distal end 17 of rod 10 nearest as possible to anchor 2, especially after having introduced distal end 21 of tube 20 within sub-cortical volume 3A delimited in bone 3 where anchor 2 is implanted, as shown in figure 14. Of course, such introduction of the tube below the surface of bone 3 is possible only if the outside diameter of tube 20 is lower than the diameter of the bone hole in which anchor 2 is implanted, typically if the outside diameter of the tube is lower than the maximal outside diameter of anchor 2.

According to a not shown embodiment, the relative arrangement between distal end 21 of tube 20 and distal portion 19 of hole 12 can be reversed: rather than, as the embodiment shown in figures 1 to 14, distal end 21 of tube 20 lies perpendicularly to axis X-X and the distal end points 15A and 16A of the openings 15 and 16 are offset along this axis, it is possible to align the two radially opposed openings of the hole provided in distal end part of the rod, with the distal portion of this hole extending perpendicularly to the axis of the rod, if the two portions of the distal end part of the tube, respectively facing the two opposed sides of the rod where are delimited these openings, have respective distal end points which are offset with respect to each other along the axis of the rod.

More generally, other embodiments of the invention are possible, including by recombining the various elements disclosed herein in different or alternative combinations. Although the above description contains many specifics, these should not be considered as limiting the scope of the invention as defined by the appended claims, but as merely providing illustrations of some of the embodiments of this invention.

## Claims

1. An arthroscopic device (1) for cutting a suture, comprising:
- an elongated member (10) which defines a longitudinal axis (X-X) and which includes a distal part (11) provided with a through hole (12) joining first and second openings (15, 16) respectively defined in radially opposed first and second sides (13, 14) of the member, and
- an elongated body (20) which is assembled with the member (10) with a non-zero radial tolerance (T) and which is axially moveably disposed with respect to the member (10) between a load position, in which the body is axially away from the hole (12) and thus keeps the hole free to receive at least one suture (4) extending in length through the first and second openings (15, 16), and a cut position, in which the body axially covers in totality the first and second openings and cooperates with the hole for cutting the at least one suture (4), via an intermediate position in which, on the first side (13) of the member, the first opening (15) is kept at least partly free by the body, whereas, on the second side (14) of the member, the second opening (16) is axially covers in totality by the body,
**characterized in that** the body (20) is tubular and is moveably mounted around the member (10),
and **in that**, when the tubular body (20) is in the intermediate position with respect to the member (10), a radial gap (24), through which the at least one suture (4) can slip , is defined on the second side (14) of the member between a corresponding portion of a distal end (17) of the member and a non-cutting portion of a distal end (21) of the tubular body (20), so that, when both the tubular body is moved from the intermediate position to the cut position and the part of the at least one suture, passing through the gap, is tensioned, the gap is radially enlarged by a biasing action of the tensioned part of the at least one suture between the tubular body and the second side of the member, up to correspond to the tolerance (T), while another portion of the distal end of the tubular body, that is radially opposed to the non-cutting portion thereof, radially moves towards the first side (13) of the member (10), up to cooperate without clearance with a distal portion of the first opening (15) for cutting the at least one suture, a cutting edge being provided at said another portion of the distal end of the tubular body and/or a cutting edge being provided at said distal portion of the first opening.

2. The device according to claim 1, **characterized in that**, along the axis (X-X), the first opening (15) has a distal end point (15A) which is distal offset with respect to a distal end point (16A) of the second opening (16).

3. The device according to claim 2, **characterized in that** the distal end (21) of the tubular body (20) lies in a geometrical plane substantially perpendicular to the axis (X-X).

4. The device according to claim 1, **characterized in that**, along the axis, the distal end of the tubular body has a distal end point facing the first side of the member, which is proximal offset with respect to a distal end point of the distal end of the tubular body, facing the second side of the member.

5. The device according to claim 4, **characterized in that** the first and second openings are substantially aligned in a direction perpendicular to the axis.

6. The device according to any of the preceding claims, **characterized in that** the hole (12) is peripherically delimited by a wall of the distal part (11) of member (10), a distal portion (19) of said wall joining the first and second opening (15, 16).

7. The device according to claim 6, **characterized in that** said wall is broken by a third opening (18), said third opening being proximal offset with respect to the first and second openings (15, 16) along the axis (X, X) and being defined in a third side of the member (10) by which the first and second sides (13, 14) are linked around the member.

8. The device according to any one of claim 6, **characterized in that** said wall is continuous so that the hole is an eyelet hole.

9. The device according to any one of the preceding claims, **characterized in that** the tubular body (20) is fixed in rotation around the axis (X-X) with respect to the member (10).

10. The device according to any one of the preceding claims, **characterized in that** at least the distal part (11) of the member (10) is constituted by a cylindrical rod which is centered on the axis (X-X).

11. The device according to any one of the preceding claims, **characterized in that** the device (1) further comprises control means (31, 32) adapted to control the movement of the tubular body (20) with respect to the member (10) between successively the load position, the intermediate position and the cut position.

12. The device according to claim 11, **characterized in that** the control means include an handle (31) which is fixedly secured to a proximal part of the member (10) and an actuator (32) which transmits force and motion to the tubular body (20) with respect to the member.

13. The device according to claim 12, **characterized in that** the actuator (32) is joined in an articulated manner to the handle (31).

14. An arthroscopic surgical kit, comprising a device (1) according to any one of the preceding claims, and at least one suture (4) adapted both to be received in the hole (12) of the member (10) of the device (1) when the tubular body (20) of the device is in the load position and to remove the tolerance (T) from the member and the tubular body at the first side (13) of the member when the tubular body is moved from the load position to the cut position.

15. The kit according to claim 14, **characterized in that** the kit further comprises at least one anchor (2), which is adapted to be implanted in a bone (3) and to which the at least one suture (4) is connected, the maximal transversal dimension of the distal end (21) of the tubular body (20) of the device (1) being lower than the maximal transversal dimension of the anchor (2).

## Patentansprüche

1. Arthroskopievorrichtung (1) zum Schneiden einer Naht, umfassend:
- ein langgestrecktes Element (10), das eine Längsachse (X-X) definiert und das einen distalen Teil (11) einschließt, der mit einem Durchgangsloch (12) versehen ist, das eine erste und eine zweite Öffnung (15, 16) verbindet, die in einer radial entgegengesetzten ersten bzw. zweiten Seite (13, 14) des Elements definiert sind, und
- einen langgestreckten Körper (20), der mit dem Element (10) mit einer radialen Toleranz (T) ungleich null zusammengesetzt ist und der axial beweglich in Bezug auf das Element (10) zwischen einer Laststellung, in welcher sich der Körper axial entfernt vom Loch (12) befindet und so das Loch freihält, um wenigstens eine Naht (4) aufzunehmen, die sich längs durch die erste und die zweite Öffnung (15, 16) erstreckt, und einer Schneidstellung, in welcher der Körper axial gänzlich die erste und die zweite Öffnung bedeckt und mit dem Loch zusammenwirkt, um die wenigstens eine Naht (4) zu schneiden, über eine Zwischenstellung, in welcher auf der ersten Seite (13) des Elements die erste Öffnung (15) zumindest teilweise frei vom Körper gehalten wird, während auf der zweiten Seite (14) des Elements die zweite Öffnung (16) axial gänzlich vom Körper bedeckt ist, angeordnet ist,
**dadurch gekennzeichnet, dass** der Körper (20) rohrförmig ist und beweglich um das Element (10) angebracht ist,
und dadurch, dass, wenn sich der rohrförmige Körper (20) in der Zwischenstellung in Bezug auf das Element (10) befindet, ein Radialspalt (24), durch den die wenigstens eine Naht (4) gleiten kann, auf der zweiten Seite (14) des Elements zwischen einem entsprechenden Abschnitt eines distalen Endes (17) des Elements und einem nichtschneidenden Abschnitt eines distalen Endes (21) des rohrförmigen Körpers (20) definiert ist, sodass, wenn sowohl der rohrförmige Körper aus der Zwischenstellung in die Schneidstellung bewegt wird als auch der Teil der wenigstens einen Naht, der durch den Spalt verläuft, gespannt wird, der Spalt radial durch eine Vorspannungswirkung des gespannten Teils der wenigstens einen Naht zwischen dem rohrförmigen Körper und der zweiten Seite des Elements vergrößert wird, bis er der Toleranz (T) entspricht, während sich ein anderer Abschnitt des distalen Endes des rohrförmigen Körpers, der dem nichtschneidenden Abschnitt davon radial entgegengesetzt ist, radial zur ersten Seite (13) des Elements (10) hin bewegt, bis er ohne Spiel mit einem distalen Abschnitt der ersten Öffnung (15) zum Schneiden der wenigstens einen Naht zusammenwirkt, wobei eine Schneidkante an dem anderen Abschnitt des distalen Endes des rohrförmigen Körpers bereitgestellt wird und/oder eine Schneidkante an dem distalen Abschnitt der ersten Öffnung bereitgestellt wird.

2. Vorrichtung nach Anspruch 1, **dadurch gekennzeichnet, dass** entlang der Achse (X-X) die erste Öffnung (15) einen distalen Endpunkt (15A) aufweist, der distal in Bezug auf einen distalen Endpunkt (16A) der zweiten Öffnung (16) versetzt ist.

3. Vorrichtung nach Anspruch 2, **dadurch gekennzeichnet, dass** das distale Ende (21) des rohrförmigen Körpers (20) in einer geometrischen Ebene liegt, die im Wesentlichen senkrecht zur Achse (X-X) ist.

4. Vorrichtung nach Anspruch 1, **dadurch gekennzeichnet, dass** entlang der Achse das distale Ende des rohrförmigen Körpers einen distalen Endpunkt aufweist, welcher der ersten Seite des Elements zugewandt ist, der proximal in Bezug auf einen distalen Endpunkt des distalen Endes des rohrförmigen Körpers, welcher der zweiten Seite des Elements zugewandt ist, versetzt ist.

5. Vorrichtung nach Anspruch 4, **dadurch gekennzeichnet, dass** die erste und die zweite Öffnung im Wesentlichen in einer zur Achse senkrechten Richtung ausgerichtet sind.

6. Vorrichtung nach einem der vorhergehenden Ansprüche, **dadurch gekennzeichnet, dass** das Loch (12) im Umfang durch eine Wand des distalen Teils (11) des Elements (10) begrenzt ist, wobei ein distaler Abschnitt (19) der Wand die erste und die zweite Öffnung (15, 16) verbindet.

7. Vorrichtung nach Anspruch 6, **dadurch gekennzeichnet, dass** die Wand durch eine dritte Öffnung (18) durchbrochen ist, wobei die dritte Öffnung proximal in Bezug auf die erste und die zweite Öffnung (15, 16) entlang der Achse (X, X) versetzt ist und in einer dritten Seite des Elements (10), durch welche die erste und die zweite Seite (13, 14) um das Element verknüpft sind, definiert ist.

8. Vorrichtung nach Anspruch 6, **dadurch gekennzeichnet, dass** die Wand durchgängig ist, sodass das Loch ein Ösenloch ist.

9. Vorrichtung nach einem der vorhergehenden Ansprüche, **dadurch gekennzeichnet, dass** der rohrförmige Körper (20) drehfest um die Achse (X-X) in Bezug auf das Element (10) ist.

10. Vorrichtung nach einem der vorhergehenden Ansprüche, **dadurch gekennzeichnet, dass** wenigstens der distale Teil (11) des Elements (10) durch eine zylindrische Stange ausgebildet ist, die um die Achse (X-X) zentriert ist.

11. Vorrichtung nach einem der vorhergehenden Ansprüche, **dadurch gekennzeichnet, dass** die Vorrichtung (1) ferner Steuermittel (31, 32) umfasst, die dazu ausgelegt sind, die Bewegung des rohrförmigen Körpers (20) in Bezug auf das Element (10) zwischen nacheinander der Laststellung, der Zwischenstellung und der Schneidstellung zu steuern.

12. Vorrichtung nach Anspruch 11, **dadurch gekennzeichnet, dass** die Steuermittel einen Griff (31), der fest an einem proximalen Teil des Elements (10) angebracht ist, und ein Betätigungselement (32), das Kraft und Bewegung auf den rohrförmigen Körper (20) in Bezug auf das Element überträgt, einschließen.

13. Vorrichtung nach Anspruch 12, **dadurch gekennzeichnet, dass** das Betätigungselement (32) gelenkig mit dem Griff (31) verbunden ist.

14. Chirurgisches Arthroskopiekit, umfassend eine Vorrichtung (1) nach einem der vorhergehenden Ansprüche und wenigstens eine Naht (4), die dazu ausgelegt ist, sowohl in dem Loch (12) des Elements (10) der Vorrichtung (1) aufgenommen zu werden, wenn sich der rohrförmige Körper (20) der Vorrichtung in der Laststellung befindet, als auch die Toleranz (T) von dem Element und dem rohrförmigen Körper auf der ersten Seite (13) des Elements zu entfernen, wenn der rohrförmige Körper aus der Laststellung in die Schneidstellung bewegt wird.

15. Kit nach Anspruch 14, **dadurch gekennzeichnet, dass** das Kit ferner wenigstens einen Anker (2) umfasst, der dazu ausgelegt ist, in einem Knochen (3) implantiert zu werden, und mit dem die wenigstens eine Naht (4) verbunden ist, wobei die maximale Querabmessung des distalen Endes (21) des rohrförmigen Körpers (20) der Vorrichtung (1) kleiner als die maximale Querabmessung des Ankers (2) ist.

## Revendications

1. Dispositif arthroscopique (1) pour couper une suture, comprenant :
- un élément allongé (10) qui définit un axe longitudinal (X-X) et qui comprend une partie distale (11) pourvue d'un trou traversant (12) joignant des première et deuxième ouvertures (15, 16) respectivement définies dans des premier et deuxième côtés (13, 14) radialement opposés de l'élément, et
- un corps allongé (20) qui est assemblé avec l'élément (10) avec une tolérance radiale non nulle (T) et qui est disposé de manière axialement mobile par rapport à l'élément (10) entre une position de charge, à laquelle le corps est espacé axialement du trou (12) et maintient ainsi le trou libre de recevoir au moins une suture (4) s'étendant en longueur à travers les première et deuxième ouvertures (15, 16), et une position de coupe, à laquelle le corps recouvre axialement en totalité les première et deuxième ouvertures et coopère avec le trou pour couper ladite au moins une suture (4), via une position intermédiaire à laquelle, sur le premier côté (13) de l'élément, la première ouverture (15) est maintenue au moins partiellement libre par le corps, tandis que, sur le deuxième côté (14) de l'élément, la deuxième ouverture (16) est recouverte axialement en totalité par le corps,
**caractérisé en ce que** le corps (20) est tubulaire et est monté de manière mobile autour de l'élément (10),
et **en ce que**, lorsque le corps tubulaire (20) est à la position intermédiaire par rapport à l'élément (10), un espace radial (24), à travers lequel ladite au moins une suture (4) peut glisser, est défini sur le deuxième côté (14) de l'élément entre une partie correspondante d'une extrémité distale (17) de l'élément et une partie non de coupe d'une extrémité distale (21) du corps tubulaire (20), de sorte que, lorsque, à la fois, le corps tubulaire est déplacé de la position intermédiaire à la position de coupe et la partie de ladite au moins une suture, passant à travers l'espace, est sous tension, l'espace est radialement agrandi par une action de sollicitation de la partie sous tension de ladite au moins une suture entre le corps tubulaire et le deuxième côté de l'élément, jusqu'à ce qu'il corresponde à la tolérance (T), tandis qu'une autre partie de l'extrémité distale du corps tubulaire, qui est radialement opposée à la partie non de coupe de celle-ci, se déplace radialement vers le premier côté (13) de l'élément (10), jusqu'à ce qu'elle coopère sans jeu avec une partie distale de la première ouverture (15) pour couper ladite au moins une suture, un bord de coupe étant prévu au niveau de ladite autre partie de l'extrémité distale du corps tubulaire et/ou un bord de coupe étant prévu au niveau de ladite partie distale de la première ouverture.

2. Dispositif selon la revendication 1, **caractérisé en ce que**, le long de l'axe (X-X), la première ouverture (15) a un point d'extrémité distale (15A) qui est décalé de manière distale par rapport à un point d'extrémité distale (16A) de la deuxième ouverture (16).

3. Dispositif selon la revendication 2, **caractérisé en ce que** l'extrémité distale (21) du corps tubulaire (20) se trouve dans un plan géométrique sensiblement perpendiculaire à l'axe (X-X).

4. Dispositif selon la revendication 1, **caractérisé en ce que**, le long de l'axe, l'extrémité distale du corps tubulaire a un point d'extrémité distale faisant face au premier côté de l'élément, qui est décalé de manière proximale par rapport à un point d'extrémité distale de l'extrémité distale du corps tubulaire, faisant face au deuxième côté de l'élément.

5. Dispositif selon la revendication 4, **caractérisé en ce que** les première et deuxième ouvertures sont sensiblement alignées dans une direction perpendiculaire à l'axe.

6. Dispositif selon l'une quelconque des revendications précédentes, **caractérisé en ce que** le trou (12) est délimité périphériquement par une paroi de la partie distale (11) de l'élément (10), une partie distale (19) de ladite paroi joignant les première et deuxième ouvertures (15, 16).

7. Dispositif selon la revendication 6, **caractérisé en ce que** ladite paroi est rompue par une troisième ouverture (18), ladite troisième ouverture étant décalée de manière proximale par rapport aux première et deuxième ouvertures (15, 16) le long de l'axe (X-X) et étant définie dans un troisième côté de l'élément (10) par lequel les premier et deuxième côtés (13, 14) sont liés autour de l'élément.

8. Dispositif selon la revendication 6, **caractérisé en ce que** ladite paroi est continue de sorte que le trou est un oeillet.

9. Dispositif selon l'une quelconque des revendications précédentes, **caractérisé en ce que** le corps tubulaire (20) est fixe en rotation autour de l'axe (X-X) par rapport à l'élément (10).

10. Dispositif selon l'une quelconque des revendications précédentes, **caractérisé en ce qu'**au moins la partie distale (11) de l'élément (10) est constituée par une tige cylindrique qui est centrée sur l'axe (X-X).

11. Dispositif selon l'une quelconque des revendications précédentes, **caractérisé en ce que** le dispositif (1) comprend en outre des moyens de commande (31, 32) conçus pour commander le mouvement du corps tubulaire (20) par rapport à l'élément (10) successivement entre la position de charge, la position intermédiaire et la position de coupe.

12. Dispositif selon la revendication 11, **caractérisé en ce que** les moyens de commande comprennent une poignée (31) qui est fixée fermement à une partie proximale de l'élément (10) et à un actionneur (32) qui transmet la force et le mouvement au corps tubulaire (20) par rapport à l'élément.

13. Dispositif selon la revendication 12, **caractérisé en ce que** l'actionneur (32) est joint d'une manière articulée à la poignée (31).

14. Kit chirurgical arthroscopique, comprenant un dispositif (1) selon l'une quelconque des revendications précédentes, et au moins une suture (4) conçue à la fois pour être reçue dans le trou (12) de l'élément (10) du dispositif (1) lorsque le corps tubulaire (20) du dispositif est à la position de charge et pour supprimer la tolérance (T) par rapport à l'élément et au corps tubulaire du premier côté (13) de l'élément lorsque le corps tubulaire est déplacé de la position de charge à la position de coupe.

15. Kit selon la revendication 14, **caractérisé en ce que** le kit comprend en outre au moins un élément d'ancrage (2), qui est conçu pour être implanté dans un os (3) et auquel ladite au moins une suture (4) est reliée, la dimension transversale maximale de l'extrémité distale (21) du corps tubulaire (20) du dispositif (1) étant inférieure à la dimension transversale maximale de l'élément d'ancrage (2).
